# EUROPEAN PATENT APPLICATION

(11) **EP 1 352 671 A1**
(43) Date of publication of application: **15.10.2003**
(21) Application number: 02007818.4
(22) Date of filing: 08.04.2002
(51) Int. Cl.: A61M 29/02, A61M 25/10

(54) **PTCA and/or PTA balloon**

(71) Applicant: Acrostak Corp., 8409 Winterthur (CH)
(72) Inventor: Stahl, Laurent, 3414 Oberburg (CH); Verin, Vitali, 1202 Genf (CH); Berger, Erwin, 9507 Stettfurt (CH); Schwager, Michael, 8404 Winterthur (CH)
(74) Representative: Weiss, Wolfgang

(57) **Abstract**

The invention describes a flexible PTCA and/or PTA balloon, which respects the curves of the anatomy for dilatation, centering and setting stents and a special method for inflation.

## Description

This invention relates to a PTCA and/or PTA balloon for a dilatation catheter, for setting stents and for centering a catheter into the vessel.

Coronary angioplasty ("PTCA") is an established treatment for coronary artery disease. Percutaneous transluminal angioplasty (PTA) is an established treatment for peripheral artery disease. The procedure involves inserting a balloon catheter, through a vascular structure-to-structure site at which atherosclerotic plaque has collected on the vessel wall. That means coronary arteries, peripheral arteries and veins, which carry blood. The plaque is disrupted by inflating the balloon at the distal end of the catheter, thereby increasing the intraluminal diameter of the vascular structure and spreading or loosening the plaque. Disruption of the plaque ultimately reduces the restriction to the blood flow through the vascular structure. After sufficient expansion, the balloon is then deflated and removed, and the area of disruption heals.

While the PTCA and/or PTA technique is very widely used, one problem, which limits its acceptability, is a possible consequence known as restenosis. In hopes of preventing abrupt closures and restenosis, the stents techniques were developed. Such stents are tubular devices, which provide structural support for maintaining a vessel open.

Procedures used for stent deployment in a vessel generally involve the introduction of a stent, in a crimped condition onto a balloon catheter into a vessel and the optimal localisation of the stent relative to the intended implantation or target site, followed by the expansion of the stent through inflation of the balloon such that is locked in the desired position in apposition to the vessel wall. Certains stents require an ancillary means for expansion. For example, a stent may fitted over a deflated angioplasty balloon, which is then introduced into the vessel and inflated, thereby expanding the stent and deploying it correctly. The procedure of setting stents incorporates the following steps: An adequate channel for passage of the balloon stent assembly is created by inflating a first balloon without stent within the stenosed region. Then the balloon stent assembly is advanced into the target vessel, the crimped stent is localised and optimally positioned relative to the intended implantation site in the stenosis, and the stent is expanded by inflating the "carrier" balloon, so as to achieve contact between the stent and the walls of the vessel. After that step the axially symmetric tubular geometry of the stent and uniform circumferential contact of the stent with the walls of the vessel were optimised by inflating another balloon capable of withstanding relatively high distending pressures within the deployed stent. In order to avoid damage to the target vessel adjacent to implanted stent, the balloon used for post-dilatation is optimally of the same length or shorter than the stent.

The patent US 5,213,361 describes a device for preventing restenosis after angioplasty comprising, among various embodiments, a catheter having a balloon at its distal end and a centre core or tube in which a conventional guide wire is receivable. Particles or crystals of radioactive material are embedded in or mounted on the tube inside the balloon and a retractable shielding sleeve is slidable along the tube to cover the radioactive source, blocking exposure to radiation until it is shifted away. Such a structure is said to allow radiation of a vascular structure immediately following completion of angioplasty, without separately inserting a radiation source.

Standard dilatation balloons are not well-suited transport and to take up radioactive radiating sources because the centre core or guide wire lumen tends to warp on the stretch inside the balloon, thereby forming an undulated line. The radioactive radiation source, however, has to be centred as exactly as possible inside the vessel in order to avoid the vessel wall being burned.

The document US 5,976,106 describes a balloon catheter comprising a catheter tube surrounded distally by an elongated inflatable balloon. Throughout the catheter tube is a longitudinal lumen for positioning a radioactive radiation emitter within the balloon. The catheter comprising a second lumen for directing inflation fluid into the balloon. Waist means creating a waist are mounted on the balloon to squeeze it down to nearly the diameter of the catheter thereby leaving a small passage for the inflation fluid. The waist means divide the balloon into sections to assure a close centre fit of the catheter within the balloon.

The document US 5,910,101 discloses an intravascular catheter having an expandable inflation region adapted for centering a radiation dose in a body lumen for a period of time sufficient to permit delivery of a radiation dose to the body lumen. The catheter including a delivery lumen, and a blind internal lumen for receiving a wire having a radiation source located at the distal end of the wire. The blind internal lumen is received in the delivery lumen of the catheter. The blind internal lumen prevents contamination of the radiation source by fluids from the body lumen. The radiation source is advanced through the blind internal lumen towards the inflation region of the catheter. The inflation region includes a plurality of balloon lobe at the distal end of the catheter. The plurality of balloon lobe, when inflated, can centre the radiation source within a curved section of the body lumen.

The purpose of this invention is to provide a PTCA and/or PTA balloon with a sufficient flexibility for use in sinuous coronary arteries for PTCA balloon or sinuous peripheral arteries for a PTA balloon. The balloon is after inflation enough flexible and follows during the placement the largest and/or smallest curvatures. The invention provides very short balloons with a waist for preventing a slippery effect (which is the typical reaction of a known short balloon) during the inflation phase of the balloon. The shorter the balloon the smaller is the section of the vessel inner wall damage. The invention gives a possibility for a ideal positioning in a stenosis of the coronary and/or peripheral arteries.

For solving these problems the claims give the solution.

The known meaning of the term "compliance" is the functional relation between the pressure inside the balloon and the outer diameter of the balloon.
The meaning in the invention of the term "compliance" is the relation between the momental pressure in the balloon and its momental diameter during inflation.

The invention makes possible depending on the use of the inventive balloon to adjust the compliance. This caracteristic reduces the damaging of the vessel innerwall and gives an anti-slippery effect to the balloon: A more precise location by the stenosis is attainable.

The PTCA and/or PTA inflation balloon has at least one balloon lobe. When the balloon has more than one lobe then at least one waist is situated between the lobes. The lobes and the waists are made from plastic. No means (for example rings etc) are necessary for making the waists. After the inflation process the ends of the balloon near the waists don't have any fold, which is a disadvantage in the state of the art. Preferably the balloon has 2 till 5 lobes in line. The usual total length of the inventive PTCA and/or PTA inflation balloon is in the range of 6 mm to50 mm, preferably from 8 to 40. The length of a balloon lobe is in the range of 3 mm to 40 mm, preferably from 4 to 10mm. The invention creates an inline of balloons, which total length is either longer or shorter than balloons known in the prior art. In the state of inflation the balloon remains flexible along a vessel curvature or sinuosity.

The PTCA inflation balloon having a diameter of the lobe in the range from 1 mm to 5 mm, preferably from 2 mm to 4 mm. The diameter of the waist is in the range from 1 mm to 2 mm, preferably from 1.1 mm to 1.5 mm. The PTCA inflation balloon is working at a pressure range from 4 atm till 30 atm, preferably from 5 atm to 15 atm.

The PTA inflation balloon having a diameter of the lobe in the range from 1 mm to 30 mm, preferably from 2 to 15 mm. The diameter of the waist is in the range from 1 mm to 5 mm, preferably from 1.1 to 4.5 mm. The PTA inflation balloon is working at a pressure range from 4 atm till 30 atm, preferably from 5 atm to 25 atm.

The diameters of the single lobe inside the balloon can be different. For some PTCA and/or PTA balloons it is necessary that the balloon lobes have different sizes. The distal balloon lobe is smaller and/or larger than the proximal balloon lobe.

To achieve a right positioning of the balloon at the plaque location, the balloon could have at least two knobs. The knobs are arranged near both ends of the balloon . Knobs are preferably arranged along the circumference of a balloon .

The method for inflating the PTCA and/or PTA inflation balloon has at least two phases. For example, in the first phase of the balloon inflation the waist does not inflate and dilate and has an anti-slippery effect ( because the stenosis will be blocked into the waist of the balloon), in the second phase the waist will disappear during the further balloon inflation preferably at pressure from 8 atm to 12 atm.

The non slippery effect due to knobs placed onto the balloon is ideal when using a stent balloon assembly. The stent crimped onto the balloon is better fixed on the balloon through the knobs.

The PTCA and/or PTA inflation balloon is preferably claimed appropriate for dilatation, bifurcation dilatation, centering, stenting, osteal stenting, bifurcation stenting applications.

Additional objects and features of the invention will appear from the following description in which the preferred embodiments are set forth in detail in conjunction with the accompanying drawings.

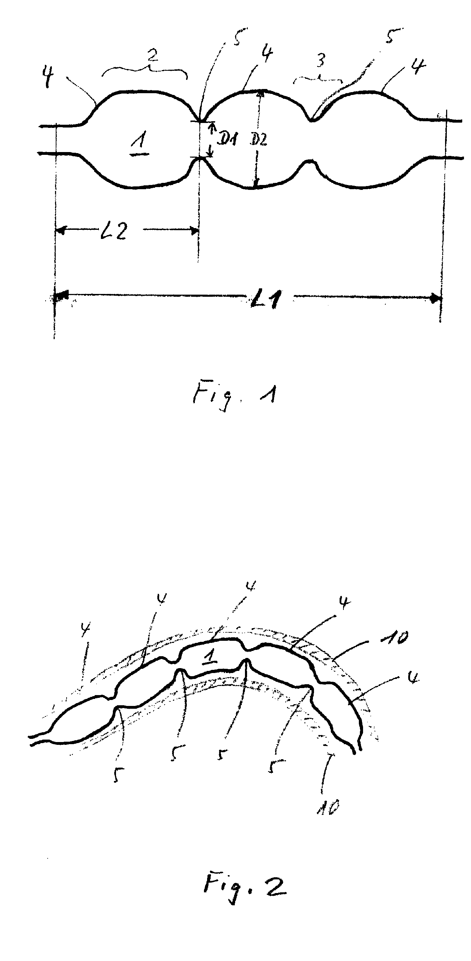

Figure 1 is a longitudinal cut of a possible PTCA dilatation balloon.

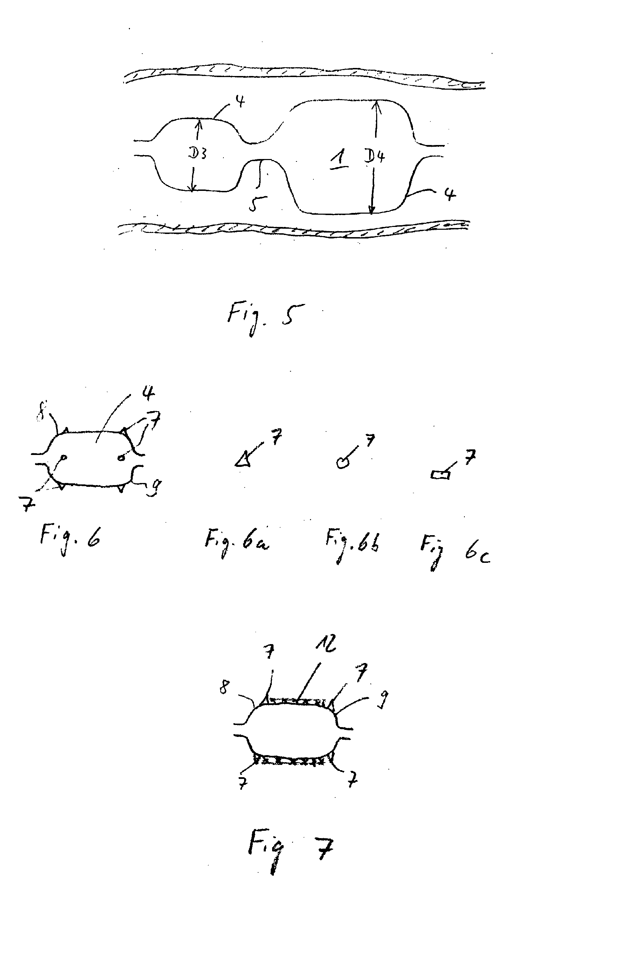

The balloon 1 has three balloon lobes 4 with two waists 5. The regions 2 the lobe and the regions 3 the waist have a different compliance as described before and shown in figure 3. D1 is the outer diameter of the waist and D2 is the outer diameter of the whole balloon 1 or the balloon lobe 4. The length L1 is the total length of the whole balloon 1 and L2 is the length of the balloon lobe 4.

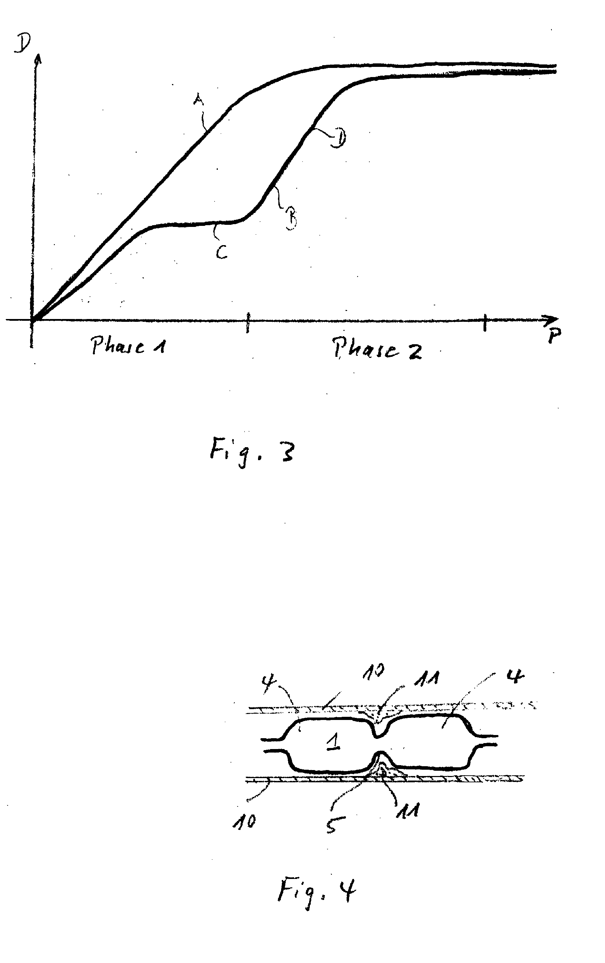

Figure 2 shows an assembly of a PTCA balloon 1 with five lobes 4 and four waists 5 in line along a vessel 10 like arteries. The balloon is made for curved and sinuous vessels 10.

The diagram in figure 3 explains the term "compliance", that means the relation between the momental pressure in the balloon and its momental diameter during inflation. The curve A shows a normal behaviour of a balloon known from the prior art. The diagram B shows a typical two phase method for inflating the PTCA and/or PTA inflation balloon. In the plateau C of phase 1 the waist does not inflate and dilate and has an anti-slippery effect, in phase 2 the waist will disappear during the further balloon inflation D preferably at pressure from 8 atm to 12 atm. This behaviour reduces from damage of the vessel inner walls . Furthermore, the invention makes possible a more precise location of the waist exactly at the plaque/stenosis of the vessel 10.

Figure 4 shows a PTCA and/or a PTA balloon 1 with two lobes 4 which are situated near a position at the plaque 11 in a vessel 10. The central waist 5 will first have an anti-slippery effect at low pressure (for example under about 8 atm till 10 atm) and will disappear while inflating the balloon 1 with more pressure, like shown in figure 3.

A special application of a PTCA balloon 1 is shown in Figure 5. The balloon 1 has two lobes 4 with different diameters D3 and D4.This type of balloon 1 is ideal for bifurcation dilatation

Knobs 7 prevent the balloon to slipper out of the stenosis. One lobe 4 is arranged with knobs 7. Knobs 7 can be differently shaped as shown in the figures 6a, 6b and 6c. The knobs 7 could be situated at many different places on the surface of the balloon lobe 4, e.g. two knobs 7 are arranged at the ends 8 and 9 and/or knobs are arranged around the circumference of the lobe 4. All possibilities are possible. The embodiment that we call knobs 7 is shown in the figures 6, 6a, 6b and 6c.

Figure 7 shows a lobe of a stent balloon assembly. The stent 12 is arranged between the knobs 7. The configuration of knobs 7 secures the stent during the movement of the stent balloon assembly within the vessel.

## Claims

1. PTCA and/or PTA inflation balloon (1) for a catheter having at least one regions (2, 3) with one defined adjustable compliance.

2. PTCA and/or PTA inflation balloon (1) for a catheter having at least two regions (2, 3) with at least two different defined compliance.

3. PTCA and/or PTA inflation balloon (1) according to claim 2 having at least two balloon lobes with a waist (5) between the balloon lobes (4).

4. PTCA inflation balloon (1) according to claim 3 having a diameter (D1) of the waist in the range from 1 mm to 2 mm, preferably 1.1 mm to 1.5 mm.

5. PTA inflation balloon (1) according to claim 3 having a diameter (D1) of the waist in the range from 1 mm to 5 mm, preferably 1.1 mm to 4.5 mm.

6. PTCA inflation balloon (1) according to claim 3 and 4 having a diameter (D2) of the lobes (4) in the range from 1 mm to 5 mm, preferably 2 mm to 4 mm.

7. PTA inflation balloon (1) according to claim 3 and 5 having a diameter (D2) of the lobes (4) in the range from 1 mm to 30 mm, preferably 2 mm to 15 mm.

8. PTCA and/or PTA inflation balloon (1) according to at least one of the previous claims wherein the diameters (D3, D4) of the balloon lobes (4) are different.

9. PTCA and/or PTA inflation balloon (1) according to at least one of the previous claims having a total length (L1) in the range 6 mm to 50 mm, preferably 8 mm to 40 mm.

10. PTCA and/or PTA inflation balloon (1) according to at least one of the previous claims wherein the balloon lobes (4) having a length (L2) in the range of 3 mm to 40 mm, preferably 4 mm to 10 mm.

11. PTCA and/or PTA inflation balloon (1) according to at least one of the previous claims having 2, 3, 4 or 5 balloon lobes (4) in line.

12. PTCA and/or PTA inflation balloon (1) according to at least one of the previous claims having at least two knobs (7).

13. Method for inflating the PTCA and/or PTA inflation balloon (1) claimed with the previous claims having at least two phases for inflating the balloon.

14. PTCA and/or PTA inflation balloon (1) according to at least one of the previous claims used for dilatation, bifurcation dilatation, centering, stenting, osteal stenting, bifurcation stenting applications.
